# EUROPEAN PATENT SPECIFICATION

(11) **EP 2 105 095 B1**
(45) Date of publication and mention of the grant of the patent: **08.05.2019**
(21) Application number: 09004455.3
(22) Date of filing: 27.03.2009
(51) Int. Cl.: A61B 17/00

(54) **Treatment instrument for endoscopic use**
Behandlungsinstrument zur endoskopischen Anwendung
Instrument de traitement pour utilisation endoscopique

(30) Priority: 28.03.2008 US 57839
(43) Date of publication of application: 30.09.2009
(73) Proprietor: Olympus Corporation, Tokyo 192-8507 (JP)
(72) Inventor: Nomura, Yusuke, Hachioji-shi, Tokyo 192-8507 (JP); Takahashi, Misa, Hachioji-shi, Tokyo 192-8507 (JP); Miyano, Hiromichi, Hachioji-shi, Tokyo 192-8507 (JP); Katada, Keisuke, Hachioji-shi, Tokyo 192-8507 (JP)
(74) Representative: von Hellfeld, Axel

(56) References cited:
- EP-A1- 1 491 153
- GB-A- 2 321 193
- US-A- 2 839 049
- US-A- 5 501 694

## Description

### BACKGROUND OF THE INVENTION

### Field of the Invention

The present invention relates to, for example, a treatment instrument for combined use with a flexible endoscope inserted into a body cavity.

### Background Art

Some conventional treatment instruments for endoscopic use used and inserted into an operation channel of a flexible endoscope and having various treatment sections, e.g. forceps cups or incision instruments fixed to the proximal end of a flexible sheath treat tissue by operating the treatment section with an operation section provided to the distal end of the sheath. Various mechanisms for transferring a force applied to the operation section further to the treatment section have been contemplated for these treatment instruments for endoscopic use.

Japanese Unexamined Patent Application, First Publication No. 2000-229084 discloses an example of treatment instrument for endoscopic use having an operation wire inserted in the inner periphery of a sheath having a tightly-wound stainless steel wire inserted into or retracted from a channel in which, drawing the operation wire proximally causes forceps cups to make opening and closing movements via a link mechanism connected to the distal end of the operation wire. The sheath in full length is formed by a piece of wire which is wound in the axial line direction of the sheath. The wire is overlaid in two layers in the radial direction of the sheath in which the winding direction opposes between the inner layer and the outer layer. (Hereinafter called, for example, "two-layer-single-thread coil", since this naming indicates the number of layer of the same type of coil layered in the radial direction of the sheath; and the number of threads of the coil forming each layer in this order.)

In addition, Japanese Unexamined Patent Application, First Publication No. H3-47246 uses three types of coils so that the strength of the sheath increases from the distal end to the base end of the sheath gradually, and so that the ends of each adjacent coils are connected by a connecting pipe. That is, a single-layer-single-thread coil is used as a distal end coil provided in the vicinity of the distal end of the sheath; a single-layer-single-thread coil having a diameter greater than that of the distal end coil is used as an intermediate coil; and a single-layer-multithread coil is used as a rear end coil provided in the vicinity of the base end of the sheath.

US 5,501,694 discloses an expandable intravascular occlusion material removal device comprising a drive shaft composed of an inner drive shaft and an outer drive shaft. Each of the inner and outer drive shafts comprises two oppositely wound helices. A multi-filar coil may be used, specifically, three wires may be wound simultaneously to increase the winding angle of the helices.

GB 2 321 193 discloses an endoscopic treatment tool comprising a sheath formed of a short section of meshed or net pipe formed by braiding fine stainless wires, and heat-welding the pipe between tubes made from synthetic resin.

EP 1 491 153 discloses an insertion tube comprising a short-wind-pitch coil made of steel wire, and may be made of a multiple winding coil made by winding a plurality of steel lines.

In some problematic cases, however, operating a treatment section by pushing an operation wire toward the distal end causes tensile load acting on a sheath including coils to extend the sheath toward the distal end, thereby preventing transmission of force applied to the operation section and failing to obtain sufficient degree of protrusion. Also, another case encounters a drawback in which a soft sheath bends inevitably in an attempt upon catching a tissue with the treatment section to move an treatment section in the direction orthogonal to the axial line direction, thereby failing to move the treatment section to a sufficient degree.

### SUMMARY OF THE INVENTION

The present invention was conceived in consideration of the aforementioned circumstance, and an object thereof is to provide a treatment instrument for endoscopic use provided with a sheath having reduced deformability against a tensile load.

The present invention is a treatment instrument for endoscopic use including a cylindrical flexible sheath in which at least a multithread coil is used to the sheath in full length.

### BRIEF DESCRIPTION OF DRAWINGS

FIG. 1 is a cross-sectional view showing the distal end of a treatment instrument for endoscopic use according to a first embodiment of the present invention in an enlarged view.
FIG. 2 is a plan view of a proximal end section of the treatment instrument for endoscopic use according to the first embodiment of the present invention.
FIG. 3 is a perspective view of one of jaw sections according to the first embodiment of the present invention.
FIG. 4 is a perspective view of the other jaw sections according to the first embodiment of the present invention.
FIG. 5 is a cross-sectional view showing a pair of jaw sections having a projecting shape disposed corresponding to a recessed shape according to the first embodiment of the present invention.
FIG. 6 is an enlarged cross-sectional view showing the distal end section of the treatment instrument for endoscopic use in detail according to the first embodiment of the present invention.
FIG. 7 is a view showing a step of assembling a rotational ring according to the first embodiment of the present invention.
FIG. 8 is a cross sectional view taken along the line A-A in FIG. 2.
FIG. 9 is a cross sectional view taken along the line B-B in FIG. 2.
FIG. 10 is a cross sectional view taken along the line C-C in FIG. 2.
FIG. 11 is an enlarged cross-sectional view showing the distal end section of the treatment instrument for endoscopic use in detail according to a modified example of the first embodiment of the present invention.
FIG. 12 is a detailed cross-sectional view showing the vicinity of the distal end of a treatment instrument for endoscopic use according to a second embodiment of the present invention.
FIG. 13 is a detailed cross-sectional view showing the distal end of a treatment instrument for endoscopic use according to a third embodiment of the present invention.
FIG. 14 is a detailed cross-sectional view of the distal end of a modified example of the embodiment of the present invention.
FIG. 15 is a view explaining action of a modified example of the embodiment of the present invention.
FIG. 16 is a perspective view of one of the jaw sections according to a modified example of the embodiment of the present invention.
FIG. 17 is a perspective view of the other jaw sections according to a modified example of the embodiment of the present invention.
FIG. 18 is a cross-sectional view showing a pair of jaw sections having a projecting shape disposed corresponding to a recessed shape according to a modified example of the embodiment of the present invention.
FIG. 19 is a perspective view of one of the jaw sections according to a modified example of the embodiment of the present invention.
FIG. 20 is a perspective view of the other jaw sections according to a modified example of the embodiment of the present invention.
FIG 21 is a cross-sectional view showing a pair of jaw sections having a projecting shape disposed corresponding to a recessed shape according to a modified example of the embodiment of the present invention.
FIG. 22 is a view showing a step of assembling a rotational ring according a modified example of the embodiment of the present invention.
FIG. 23 is detailed a cross-sectional view of a basket according to the embodiment of the present invention.
FIG. 24 is a detailed cross-sectional view of a snare according to the embodiment of the present invention.
FIG. 25 is a detailed cross-sectional view of an incision instrument according to the embodiment of the present invention.
FIG. 26 is a detailed cross-sectional view of a syringe instrument according to the embodiment of the present invention.

### PREFERRED EMBODIMENTS

Each embodiment of a treatment instrument for endoscopic use associated with a case which the treatment instrument for endoscopic use is a forceps for endoscopic use according to the present invention will be explained as follows. It should be noted that the treatment instrument for endoscopic use not limited to a forceps for endoscopic use may be an incision instrument, etc., for endoscopic use.

### [First Embodiment]

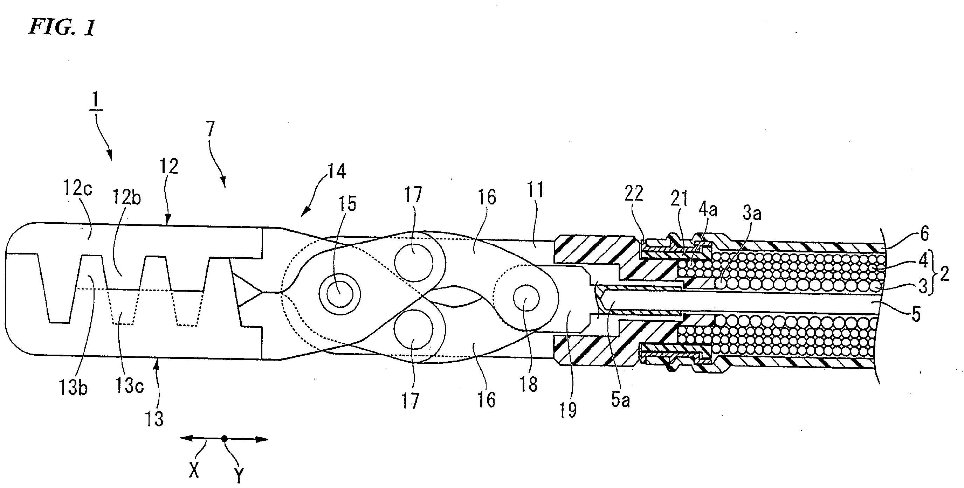

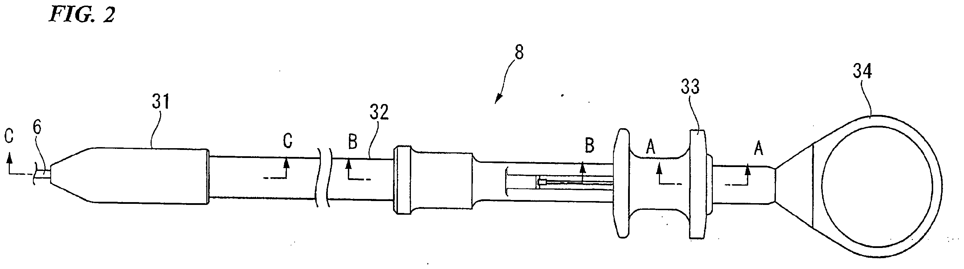

A forceps for endoscopic use according to a first embodiment of the present invention will be explained as follows with reference to FIGS. 1 to 10. FIG. 1 is a detailed cross-sectional view showing a distal end section of a forceps for endoscopic use. FIG. 2 is a plan view of a proximal end section of the forceps for endoscopic use.

As shown in FIGS. 1 and 2, a sheath 2 of a forceps 1 for endoscopic use according to the present embodiment is configured by two types of sheath overlaying in the radial direction, i.e., a first sheath 3 formed by a multithread coil disposed inward and a second sheath 4 formed by a multilayer-single-thread coil disposed outward.

The forceps 1 for endoscopic use is provided with: a cylindrical and flexible first sheath 3 and a cylindrical and flexible second sheath 4; an operation wire (elongated member) 5 inserted in the vicinity of an inner periphery of the first sheath 3; an overtube 6 surrounding the second sheath 4 from the outside in the radial direction; a treatment section 7 connected to a distal end section 3a of first sheath, a distal end section 4a of second sheath, and a distal end section 5a of operation wire for conducting treatment act; and an operation section 8 for extending and retracting the operation wire 5 relative to the first sheath 3 and the second sheath 4 in the axial line direction. The forceps 1 for endoscopic use inserted into an operation channel and entered into a body cavity reaches to a desirable tissue while bending in accordance with geometry surrounding thereof. It should be noted that a multithread coil mentioned here indicates a coil having 2 to 20 threads densely wound in the axial line direction around a predetermined axial line so that a layer is formed in the direction orthogonal to the axial line..

In addition, an example of the multithread coil used in the present embodiment is a single-layer-nine-thread coil; and an example of the multilayer-single-thread coil used in the present embodiment is a three-layer-single-thread coil. In addition, the first sheath 3 and the second sheath 4 are disposed to the full length of sheath 2, i.e. from the treatment section 7 to the operation section 8.

As shown in FIG. 1, the treatment section 7 has: a cylindrical base 11 fixed to the distal end section 3a of the first sheath 3 and the distal end section 4a of the second sheath 4 and extending in the axial line direction of the operation wire 5; and a linking mechanism 14 provided with a pair of jaw sections 12 and 13 which are a pair of members.

The linking mechanism 14 is provided with a pivotable pin 15 fixed to the base 11; and the pair of jaw sections 12 and 13 supported rotatably by the pivotable pin 15 and protruding from the base 11. In addition, the linking mechanism 14 is further provided with a pair of link plates 16, and a pin 17 supports the proximal end sections of the pair of jaw sections 12 and 13 at the distal end sections of the pair of link plates 16 respectively rotatably. Furthermore, a pin 18 supports the proximal end sections of the pair of link plates 16 rotatively at the distal end section of the connecting member 19, and the connecting member 19 is fixed to the distal end section 5a of the operation wire 5. That is, the pair of jaw sections 12 and 13 constitute a part of the linking mechanism 14.

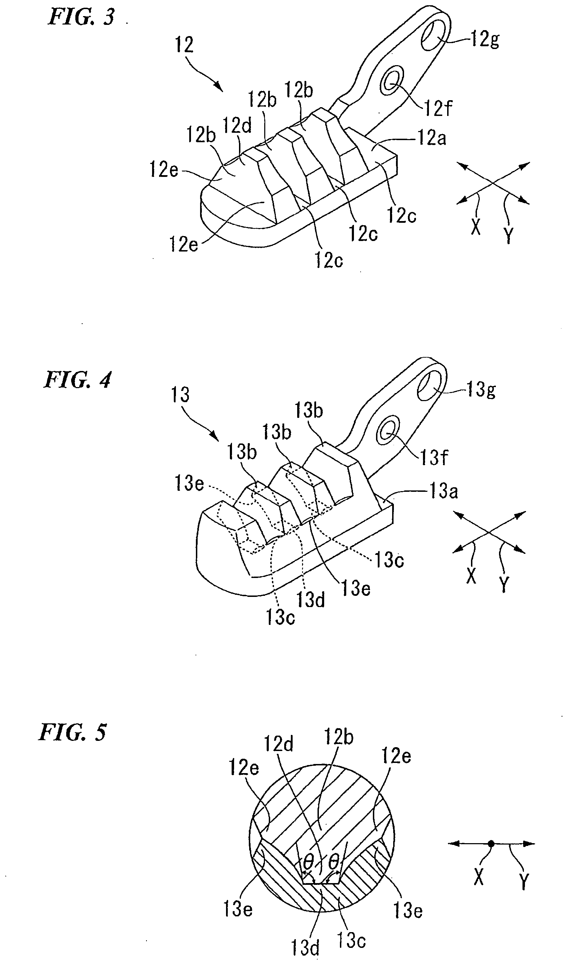

FIG. 3 shows a perspective view showing one of the jaw section 12. FIG. 4 shows a perspective view of the other one of the jaw sections 13.

Projections and depressions formed to inner surfaces 12a and 13a of the pair of jaw sections 12 and 13 respectively facing each other have corresponding shapes with respect to an axial line direction X of the operation wire 5 and intersecting the direction Y crossing with the axial line direction X. To be more specific, projecting shape 12b and recessed shape 12c are formed to the inner surface 12a of the jaw section 12; and projecting shape 13b and recessed shape 13c are formed to the inner surface 13a of the jaw section 13. It should be noted that a through hole 12f and a through hole 12g are provided in the vicinity of the proximal end of the jaw section 12; and a through hole 13f and a through hole 13g are provided in the vicinity of the proximal end of the jaw section 13. The pivotable pin 15 engaging with the through hole 12f of the jaw section 12 and the through hole 13f of the jaw section 13 supports the jaw section 12 and the jaw section 13 rotatably to each other. In addition, the jaw section 12 and the jaw section 13 are supported rotatably with respect to the link plate 16 by the pin 17 passing through the through hole 12g of the jaw section 12 and the through hole 13g of the jaw section 13.

In addition, the pair of jaw sections 12 and 13 are disposed so that the projecting shape 12b and the recessed shape 13c correspond to each other in a staggered configuration and the recessed shape 12c and the projecting shape 13b correspond to each other in a staggered configuration as shown in FIG. 1. The projecting shape 12b and the recessed shape 12c of the jaw section 12 and the projecting shape 13b and the recessed shape 13c of the jaw section 13 form the projections and depressions corresponding to each other with respect to the axial line direction X.

In addition, as shown in FIG. 3, a projection section 12d protruding from the inner surface 12a is formed to each projecting shape 12b of the jaw section 12 in the center in the intersecting direction Y of the projecting shape 12b; and shoulder sections 12e are formed to two sides of the projection section 12d in the intersecting direction Y. On the other hand, similarly, as shown in FIG. 4, a recessed section 13d of the recessed shape 13c depressing more deeply in the direction of the inner surface 13a is formed in the center in the intersecting direction Y of each recessed shape 13c of the jaw section 13; and shoulder sections 13e are formed to two sides of the recessed section 13d in the intersecting direction Y respectively.

FIG. 5 shows a cross-sectional view of the pair of jaw sections 12 and 13 in which the projecting shape 12b is disposed corresponding to the recessed shape 13c. The projection section 12d engages with the recessed section 13d, and the shoulder sections 12e engage with the shoulder sections 13e respectively in the projecting shape 12b and the recessed shape 13c. In addition, a slightly recessing and gently curved surface is formed on the outer periphery provided to the projecting shape 12b from the projection section 12d to the shoulder sections 12e.

The projection section 12d and the shoulder sections 12e of the projecting shape 12b, and the recessed section 13d and the shoulder sections 13e of the recessed shape 13c form the projections and depressions corresponding to each other with respect to the intersecting direction Y.

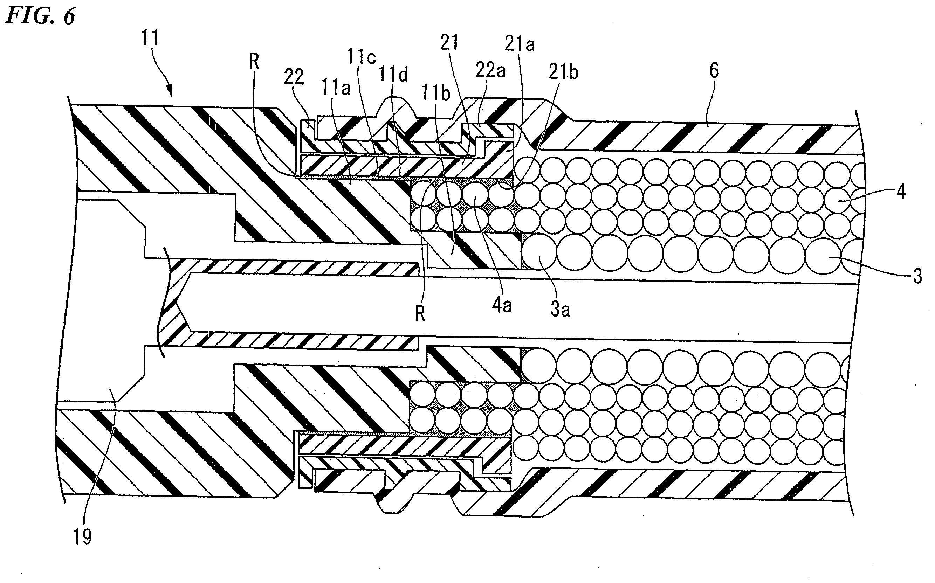

As shown in FIG. 6, a cylindrical increased-diameter section 11a is formed in the vicinity of the proximal end of the base 11; and a reduced diameter section 11b is formed more proximally relative to the increased-diameter section 11a. A cylindrical connecting pipe 21 is fixed to an outer periphery 11c of the increased-diameter section 11a by brazing method using braze R so that a part of the connecting pipe 21 is fixed to protrude toward the reduced diameter section 11b relative to a shoulder section 11d of the increased-diameter section 11a.

A cylindrical rotational ring 22 is fixed to the inner periphery of the distal end section of the overtube 6. In addition, the freely-rotatable rotational ring 22 is supported by the connecting pipe 21.

The connecting pipe 21 is provided with an engagement section 21a for preventing distal movement of the rotational ring 22. In addition, a near-link-locking section 22a corresponding to the engagement section 21a provided to the connecting pipe 21 is provided to the rotational ring 22. It should be noted that the base 11 may be provided with a near-base-locking section for supporting the freely-rotatable rotational ring 22 on the outer periphery of the base 11 and for preventing proximal movement of the rotational ring 22. In addition, it is preferable to prevent the shift of the rotational ring 22 with respect to the axial direction by disposing the near-link-locking section 22a to the proximal end section of the rotational ring 22.

The distal end section 4a of the second sheath 4 disposed between the outer periphery of the reduced diameter section 11b of the base 11 and the inner periphery of the connecting pipe is fixed to an inner periphery 21b of the connecting pipe 21 and to a surface in the vicinity of the proximal end of the increased-diameter section 11a by brazing method using braze R. Also, the distal end section 3a of the first sheath 3 is fixed to a surface in the vicinity of the proximal end of the reduced diameter section lib of the base 11 by, for example, laser welding method. That is, the distal end section 4a of the second sheath 4 is fixed to the base 11 distally relative to the distal end section 3a of the first sheath 3. It should be noted that the distal end section 4a of the second sheath 4 may be fixed at least to the inner periphery 21b of the connecting pipe 21.

Subsequently extending and retracting the operation wire 5 in the axial line direction relative to the first sheath 3 and the second sheath 4 causes the aforementioned linking mechanism 14 to vary the distance between the pair of jaw sections 12 and 13.

As previously explained, the second sheath 4 uses a three-layer-single-thread coil. The innermost layer coil and the outermost layer coil among these three-layered coils are configured to be wound in the same direction, and the intermediate-layer coil is wound in the reverse direction. According to this configuration, rotation in the direction which loosens the innermost layer and the outermost layer coil causes the intermediate layer coil to be fastened and causes the intermediate layer to interfere with the innermost layer coil, thereby transferring the rotational torque acting on the operation section 8 to the treatment section 7 in the vicinity of the distal end.

In addition, the distal end section 4a of the second sheath 4 is fixed to the inner periphery 21b of the connecting pipe 21 and to the surface in the vicinity of the proximal end of the increased-diameter section 11a by brazing method as previously explained. The brazed section of the second sheath 4 joining the adjacent coils and stiffened prevents the vicinity of the distal end of the second sheath 4 from bending thereat. This state of sheath 2 incapable of bending in the body cavity desirably reduces the operability of the forceps 1 for endoscopic use.

In addition, raw material resin used for the overtube 6 is obtained by compounding high density polyethylene and silicone oil into a flexible resin having insulation and superior expandability, e.g., low density polyethylene, polybutadiene resin, or ethylene vinyl acetate copolymer, etc. This readily reduces friction produced by the inner periphery of the overtube 6 rotating and sliding relative to the outer periphery of the second sheath 4 that are disposed inward in the radial direction of the overtube 6. Furthermore, it is revealed that manufacturing of the forceps 1 for endoscopic use necessitates the overtube 6 will be moved on the outer periphery of the second sheath 4 in the axial direction in a step for disposing the overtube 6 around the second sheath 4 fixed on the first sheath 3 previously. Friction in this case between the outer periphery of the second sheath 4 and the inner periphery of the overtube 6 can be reduced similarly.

In addition, the tolerance of the outer diameter of the first sheath 3 is used between the lower limit of 0.83 mm and the upper limit of 0.92 mm; and the tolerance of the inner diameter of the second sheath 4 is used between the lower limit of 0.92 mm and the upper limit of 0.97 mm. That is, the tolerance of clearance between the outer diameter of the first sheath 3 and the inner diameter of the second sheath 4 is set to have the lower limit of 0.00 mm and the upper limit of 0.14 mm.

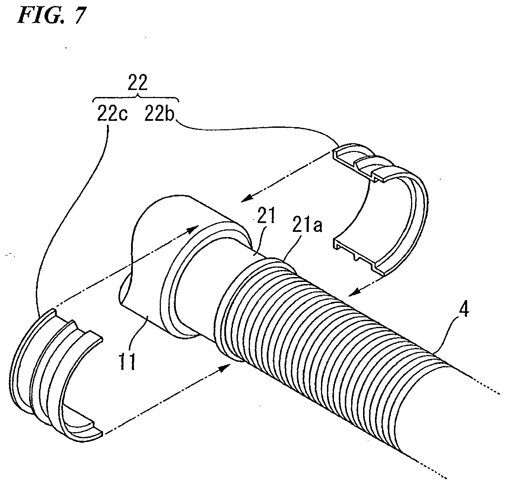

As shown in FIG. 7, the rotational ring 22 is divided longitudinally into two slices 22b and 22c in advance. It should be noted that the rotational ring 22 may be divided into three or more pieces longitudinally, or into two pieces diagonally. Subsequently, manufacturing of the forceps 1 for endoscopic use necessitates after fixing the connecting pipe 21 to the base 11 by brazing method as shown in FIG. 7, assembling the slices 22b and 22c to the outer periphery of the connecting pipe 21, and fixing the distal end section of the overtube 6 to the outer peripheries of the slices 22b and 22c.

As shown in FIG 2, the operation section 8 is provided with: a main body section 32 and a cam 31 attached to the distal end section of the main body section 32 and inserted into an operation channel of a flexible endoscope; a slider 33 for driving the treatment section 7 so that the slider 33 is capable of extending and retracting in the axial line direction and attached in the vicinity of the proximal end of the main body section 32; and a finger hook ring 34 attached to the proximal end section of the main body section 32. It should be noted that the drawings show a mere general outline since the main body section 32 uses a commonly known configuration.

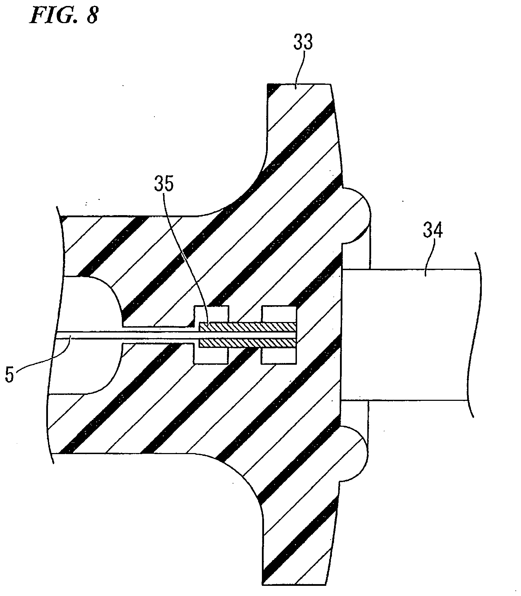

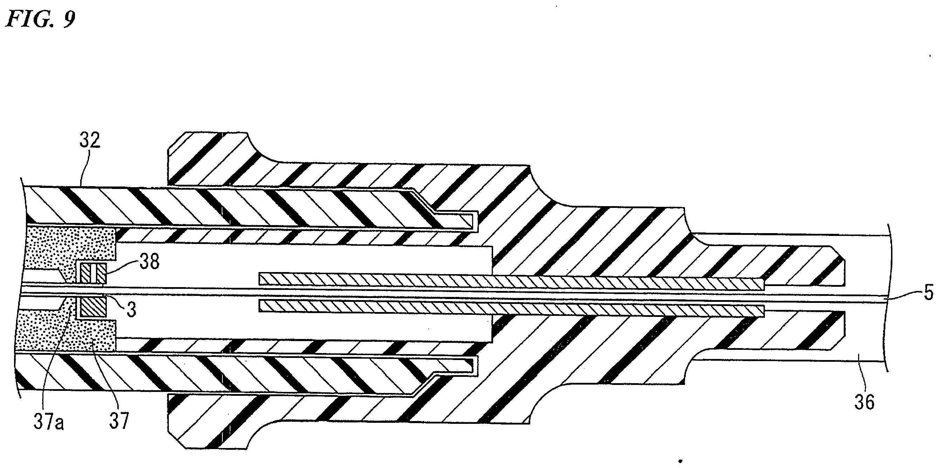

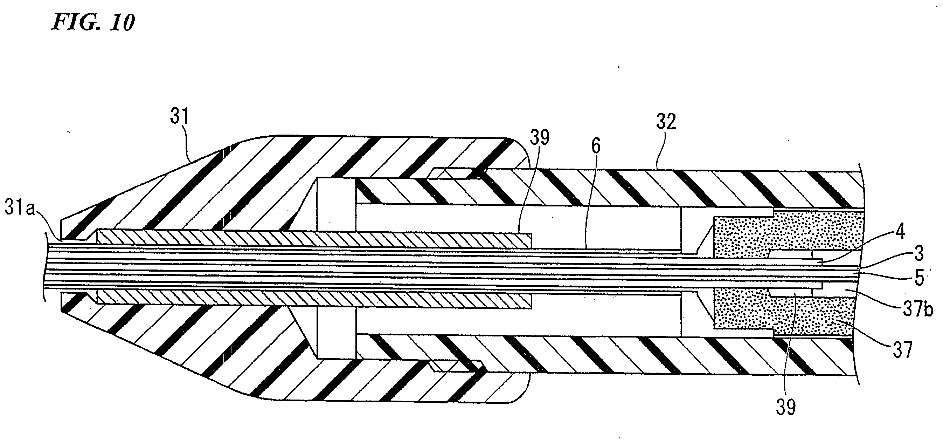

FIG. 8 is a cross sectional view taken along the line A-A in FIG. 2. As shown in FIG. 8, an engagement member 35 engages the proximal end section of the operation wire 5 to the slider 33. In addition, the operation wire 5 passing through a slit 36 formed in the main body section 32 and entering an intermediate link 37 is inserted into the first sheath 3 therein as shown in FIGS. 9 and 10. A coil receiver 38 having the proximal end of the first sheath 3 attached thereto is enclosed in the vicinity of the proximal end of the intermediate link 37. A reduced diameter section 37a preventing the distal removal of the coil receiver 38 is provided to the intermediate link 37. The first sheath 3 is inserted into the second sheath 4 in the vicinity of the distal end relative to the reduced diameter section 37a.

The second sheath 4 is attached to a coil receiver 39 by brazing method. The freely sliding coil receiver 39 is inserted into an elongated groove 37b formed to the intermediate link 37. Accordingly, the second sheath 4 engages with the intermediate link 37 in the rotational direction but in the extending and retracting direction. Furthermore, the outer periphery of the second sheath 4 extracted from the intermediate link 37 is surrounded by the overtube 6. The second sheath 4 surrounded therearound upon passing through a pipe 39 is extracted from a hole section 31a formed to the distal end of the cam 31.

Consequently, steps for carrying out operations using the forceps 1 for endoscopic use will be explained.

To start with, the treatment section 7 of the forceps 1 for endoscopic use is inserted into the body cavity via an operation channel which is not shown in the drawings. Subsequently, the treatment section 7 upon protruding the forceps 1 for endoscopic use from the distal end of the endoscope is brought close to desirable tissue in the body cavity. Undesirable directions of the pair of jaw sections 12 and 13 are adjusted by rotating the main body section 32 around the axial line to cause the pair of jaw sections 12 and 13 to rotate around the axial line. Subsequently, moving the slider 33 distally and pushing the operation wire 5 cause the pair of jaw sections 12 and 13 to open. Subsequently, abutting the pair of jaw sections 12 and 13 to the tissue to move the slider 33 distally and retract the operation wire 5 causes the tissue to be seized. Subsequently, the pair of jaw sections 12 and 13 are rotated around the axial line to dissect the desirable tissue. Also , the outer surfaces of the pair of jaw sections 12 and 13 in closed condition for removing a desirable tissue may be abutted based on tissue condition to the tissue by retracting the operation wire 5 to open the pair of jaw sections 12 and 13.

As previously explained, the base 11 is fixed to the distal end section 3a of the multithread coil, i.e., the first sheath 3 in the forceps 1 for endoscopic use according to the present embodiment. The multithread coil can receive the force for pushing or retracting the operation wire 5 by the operation section 8 in the direction close to the longitudinal direction of the wire of each coil since wires of each coil extend in the direction closer to the axial line direction of the operation wire 5 in comparison with a single-thread coil. The wire used in a coil having the most significant rigidity in its longitudinal direction can prevent the change in length of the first sheath 3 caused by applying tensile load to the first sheath 3 when the proximal end section of the operation wire 5 is pushed. In addition, the first sheath 3 densely wound in the axial line direction has significant rigidity associated with compressive load. Therefore, it is possible to prevent compressive load from being applied by retracting the proximal end section of the operation wire 5 from changing the length of the first sheath 3. Accordingly, the force for pushing or retracting the proximal end section of the operation wire 5 in the axial line direction can be transferred to the pair of jaw sections 12 and 13 of the treatment section 7 effectively.

In addition, drawback that the treatment section 7 cannot move significantly in the direction orthogonal to the axial line because of bending of the sheath 2 can be eliminated since the multithread coil used in the first sheath 3 has rigidity in the direction orthogonal to the axial line.

Also, the use of three-layer-single-thread coil in which coils adjacent to the second sheath 4 are wound in a staggered manner can cause the rotational torque acting onto the operation section 8 to be transferred to the treatment section 7 in the vicinity of the distal end, thereby improving rotational trackability of the treatment section 7.

Also, disposing the multithread coil stiffening its bending in the direction orthogonal to the axial line in an innermore side than in an outermore side can reduce the outer diameter of the multithread coil, thereby alleviating resistance in bending the first sheath 3.

In addition, the distal end section 4a of the grasping forceps 5A is fixed to the base 11 distally relative to the distal end section 3a of the first sheath 3 fixed on a surface in the vicinity of the proximal end of the reduced diameter section 11b. Accordingly, this allows the distal end section 4a of the second sheath 4 to be fixed to the base 11 in the axial direction within a range of the outer periphery of the reduced diameter section 11b. Accordingly, it is possible to prevent the second sheath 4 from stiffening in the vicinity of the distal end relative to the reduced diameter section 11b of the base 11, and it is possible to reduce the stiffening length of the distal end section 4a of the second sheath 4.

In addition, the rotational ring 22 cannot be assembled to the connecting pipe 21 distally in the axial direction since the connecting pipe 21 is provided with the engagement section 21a as shown in FIG. 6. Then, the engagement section 21a may be separated from the connecting pipe 21; the cylindrical rotational ring 22 may be assembled to the outer periphery of the connecting pipe 21; and the engagement section 21a may be attached to the connecting pipe 21 by brazing method. However, this method has drawback in which braze used in the brazing method leaking into between the rotational ring 22 and the connecting pipe 21 prevents the rotation of the rotational ring 22. The present embodiment free from drawback, in which a sliding section subject to a leak of braze used in the brazing method flowing thereinto cannot move, can provide the near-link-locking section 22a to the rotational ring 22 since the rotational ring 22 provided with the near-link-locking section 22a is divided longitudinally into the two slices 22a and 22b in prior to assembling thereof.

Aside from the aforementioned embodiment explaining the example of a configuration having the sheath 2 including two different types of sheath overlaying in the radial direction, the same effect as those in the aforementioned example can be obtained by fixing the distal end of the sheath 2 formed by the multithread coils of a common type to the inner periphery 21b of the connecting pipe 21 and to the base 11 by brazing method. In addition, it should be noted that an issue alone associated with the rotational ring 22 divided longitudinally into two or more pieces in the forceps 1 for endoscopic use will hold good as an invention.

Also, the projecting shape 12b and the recessed shape 12c of the jaw section 12 and the projecting shape 13b and the recessed shape 13c of the jaw section 13 form the projections and depressions corresponding to each other with respect to the axial line direction X. In addition, the projection section 12d and the shoulder sections 12e of the projecting shape 12b, and the recessed section 13d and the shoulder sections 13e of the recessed shape 13c form the projections and depressions corresponding to each other with respect to the intersecting direction Y. Therefore, projections and depressions formed in the axial line direction X and the intersecting direction Y can prevent the removal of seized sample in the axial line direction X and the intersecting direction Y. It should be noted that a smaller angle θ as shown in FIG. 5 can prevent the removal in the intersecting direction Y more reliably. In addition, it should be noted that an issue alone associated with the projections and depressions formed on the inner surfaces of the pair of jaw sections 12 and 13 facing each other in the forceps 1 for endoscopic use will hold good as an invention.

It should be noted that the multilayer-multithread coil having three layers used in the aforementioned second sheath 4 may be replaced by a multilayer-single-thread coil.

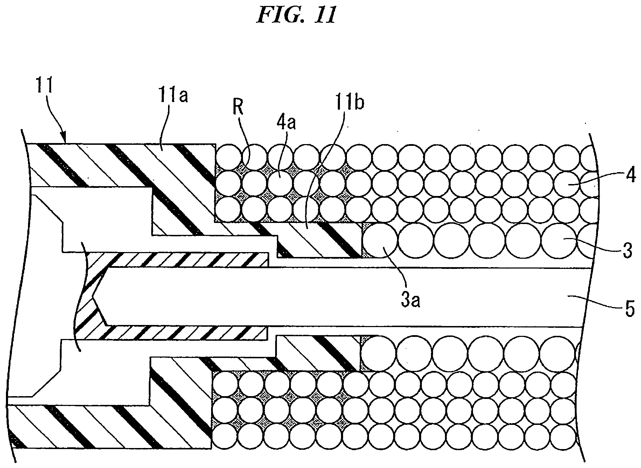

Also, a configuration as shown in FIG. 11 may be free from the overtube 6. The configuration in this case will be free from the connecting pipe 21 and the rotational ring 22. In addition, the distal end section 4a of the second sheath 4 disposed on the outer periphery of the reduced diameter section 11b of the base 11 is fixed on the outer periphery of the reduced diameter section 11b by brazing method using braze R. Also, the distal end section 3a of the first sheath 3 is fixed to a surface in the vicinity of the proximal end of the reduced diameter section 11b of the base 11 by laser welding method, etc.

Also, the second sheath 4 disposed outward may be a multilayer-multithread coil having rotational trackability as good as that of the multilayer-single-thread coil may be used in the aforementioned first embodiment.

### [First illustrative example]

A first illustrative example will be explained next. Structural elements that are equivalent in the following explanation will be assigned the same numeric symbols and redundant explanations thereof will be omitted.

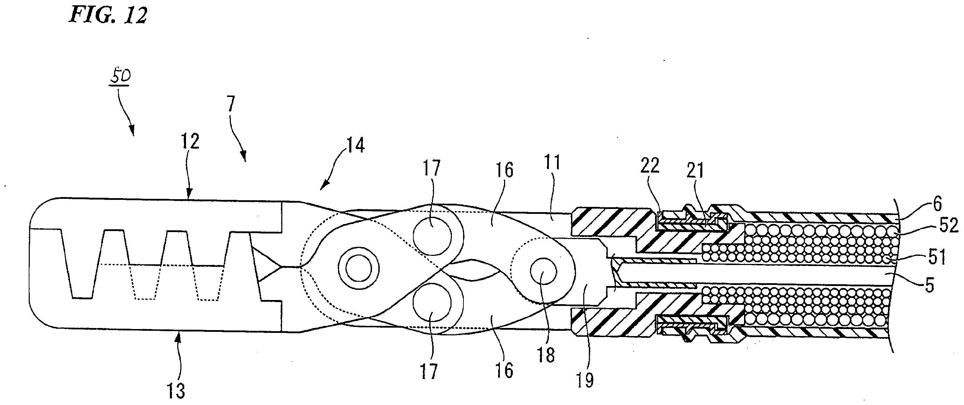

FIG. 12 is a cross-sectional view showing the distal end section of a forceps 50 for endoscopic use according to a first illustrative example. The first illustrative example is different from the first embodiment in terms of type of coils used to the first sheath and the second sheath 4. A first sheath 51 used in the first illustrative example is a three-layer-single-thread coil. A second sheath 52 used in the first illustrative example is a multithread coil. It should be noted that adjacent coils in the three-layer-single-thread coil are wound in a staggered manner; and the multithread coil is wound densely in the axial line direction.

The use of multithread coil for the second sheath 52 can prevent the change in length of the second sheath 52 caused by applying a tensile load to the second sheath 52 when the proximal end section of the operation wire 5 is pushed. In addition, the second sheath 52 densely wound in the axial line direction has significant rigidity associated with compressive load. Therefore, it is possible to prevent the compressive load from being applied to the second sheath 52 by retracting the proximal end section of the operation wire 5 from changing the length of the second sheath 52.

Also, the use of three-layer-single-thread coil in which coils adjacent to the first sheath 51 are wound in a staggered manner can cause the rotational torque acting onto the operation section 8 to be transferred to the treatment section 7 in the vicinity of the distal end, thereby improving rotational trackability of the treatment section 7.

In addition, the first sheath 51 disposed inward in the aforementioned first illustrative example may use a multilayer-multithread coil.

### [Second illustrative example]

A second illustrative example will be explained next. Structural elements that are equivalent to the aforementioned first embodiment and the first illustrative example in the following explanation will be assigned the same numeric symbols and redundant explanations thereof will be omitted.

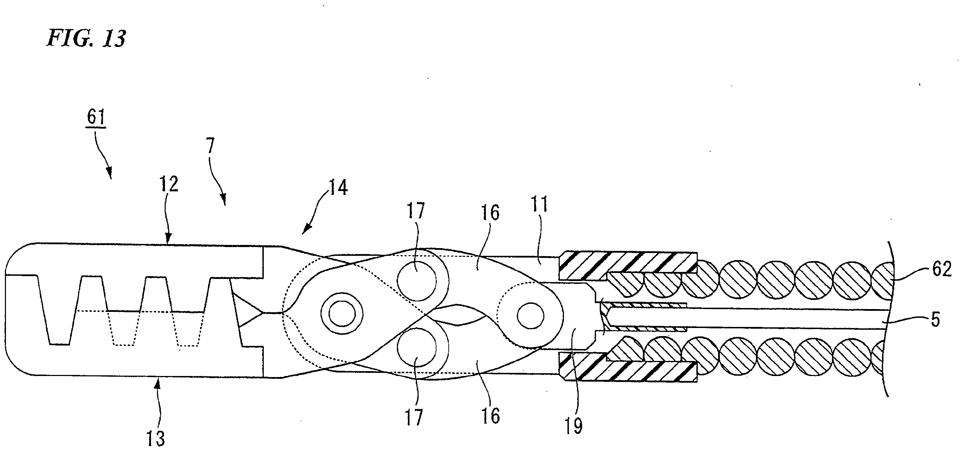

FIG. 13 is a cross-sectional view showing the distal end section of a forceps 61 for endoscopic use in detail according to a second illustrative example. The second illustrative example is different from the first embodiment in two points. Firstly, a sheath is formed by a layer of sheath 62 disposed in the radial direction. Secondly, the present invention is free from using an overtube, a rotational ring, and a connecting pipe. It should be noted that the sheath 62 uses a multithread coil having nine threads wound densely in the axial line direction.

In addition, the tolerance of the outer diameter of the operation wire 5 is used between the lower limit of 0.34 mm and the upper limit of 0.40 mm; and the tolerance of the inner diameter of the sheath 62 is used between the lower limit of 0.45 mm and the upper limit of 0.50 mm. That is, the tolerance of clearance between the outer diameter of the operation wire 5 and the inner diameter of the sheath 62 is set to have the lower limit of 0.05 mm and the upper limit of 0.16 mm.

Excessively greater clearance between the outer diameter of the operation wire 5 and the inner diameter of the sheath 62 causes the sheath 62 upon retracting the operation wire 5 to meander more significantly around the outer periphery of the operation wire 5, thereby causing a problem failing to transfer a significant degree of the force of the operation wire 5 to the treatment section 7. On the other hand, excessively smaller clearance between the outer diameter of the operation wire 5 and the inner diameter of the sheath 62 imparts more significant friction force to the operation wire 5 relative to the sheath 62, thereby deteriorating operability of the operation wire 5. The aforementioned clearance set in the second illustrative example readily provides desirable operability to the operation wire 5 and transfers the retraction force of the operation wire 5 to the treatment section 7 effectively.

The use of multithread coil for the second sheath 62 can prevent the change in length of the sheath 62 caused by applying tensile load to the sheath 62 when the proximal end section of the operation wire 5 is pushed. In addition, the sheath 62 densely wound in the axial line direction has significant rigidity associated with compressive load. Therefore, it is possible to prevent compressive load applied by retracting the proximal end section of the sheath 62 from changing the length of the sheath 62.

Accordingly, the force for extending or retracting the proximal end section of the operation wire 5 in the axial line direction can be transferred to the pair of jaw sections 12 and 13 of the treatment section 7 effectively.

Although the present invention has been described with respect to its preferred embodiments, the present invention is not limited to the embodiments described above. The configuration of the present invention allows for addition, omission, substitution and further modification without departing from the spirit and scope of the present invention.

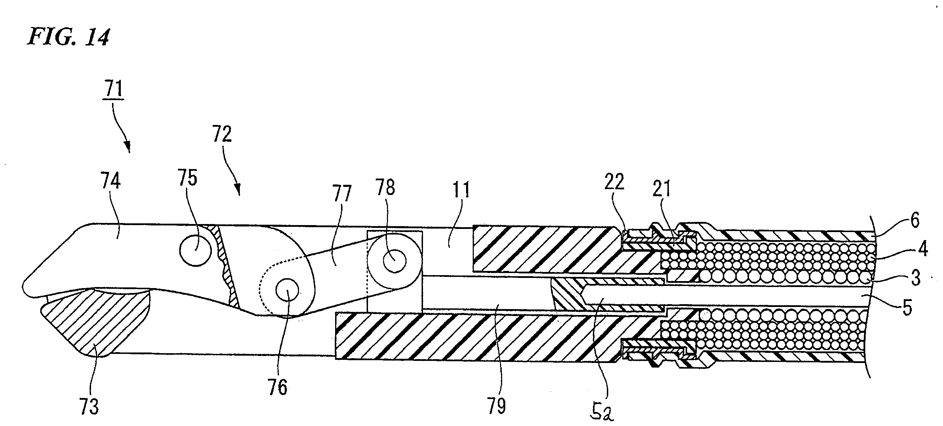

For example, the forceps 1 for endoscopic use provided with the treatment section 7 attached to the distal end of the forceps 1 for endoscopic use is explained in the aforementioned first embodiment and first and second illustrative examples. However, as shown in FIG. 14, a treatment instrument 71 for endoscopic use may be provided with a needle-holder 72 attached to the distal end section thereof.

A needle-holder 73 for enclosing a needle thereinside is fixed to an end of base 11. In addition, a pin 75 fixed to the other end of the base 11 supports a freely rotatable cover 74 around the central section thereof. A pin 76 supports the freely rotatable proximal end of the cover 74 in the vicinity of the distal end of a link plate 77. Furthermore, a pin 78 supports the proximal end of the link plate 77 rotatively at the other end of the base 11, and the connecting member 79 is fixed to the distal end section 5a of the operation wire 5. Also, the base 11 is a guideway for moving the connecting member 79 only in the axial line direction.

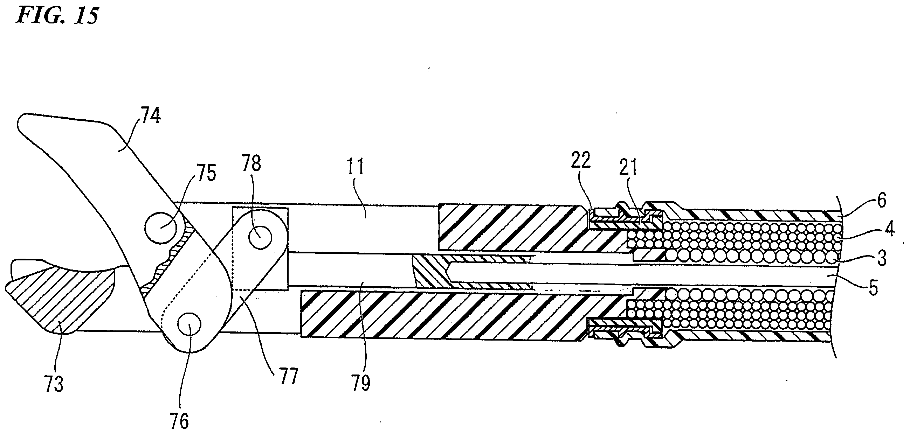

The operation of the thus configured treatment instrument 71 for endoscopic use will be explained next. Pushing this state of operation wire 5 as shown in FIG. 14 in the axial line direction causes the connecting member 79 guided by the base 11 to move distally as shown in FIG. 15. This state of pins 75, 76, and 78 causes the link plate 77 to move with rotation, thereby rotating the cover 74. The pin 75 and the pin 78 disposed on the other end of the base 11 can prevent the cover 74 and the link plate 77 from protruding partially from one end of the base 11. Accordingly, this readily reduces damage to nearby tissue which will be caused by the needle-holder 72 of the present modified example inserted into a body cavity upon extending or retracting the operation wire 5.

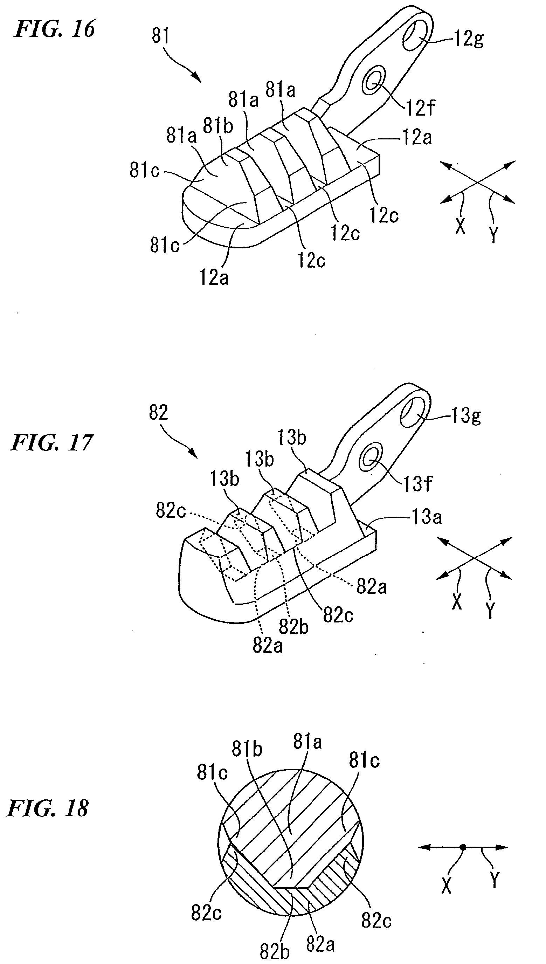

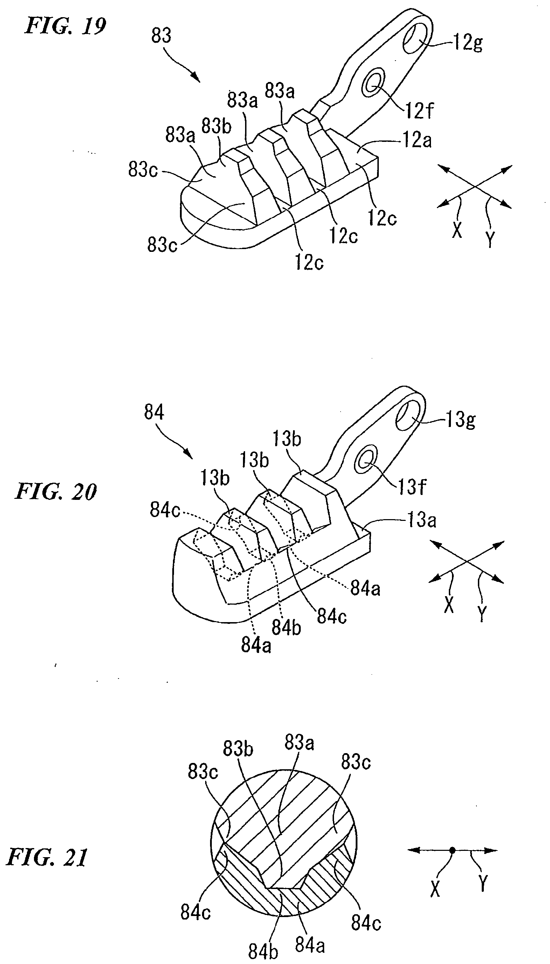

In addition, a pair of jaw sections 81 and 82 as shown in FIGS. 16 to 18 or a pair of jaw sections 83 and 84 as shown in FIGS. 19 to 21 may be provided in place of the pair of jaw sections 12 and 13 provided in the aforementioned first embodiment and first and second illustrative examples.

FIGS. 16 and 17 are perspective views showing the pair of jaw sections 81 and 82. FIG. 18 is a cross-sectional view showing the pair of jaw sections 81 and 82 disposed correspondingly. It should be noted that same components as those of the pair of jaw sections 12 and 13 in the aforementioned first embodiments will be omitted in the explanation. A projection section 81b engages with a recessed section 82b; and a shoulder section 81c engages with a shoulder section 82c as shown in FIG. 18 showing a projecting shape 81a of the jaw section 81 and a recessed shape 82a of the jaw section 82. In addition, a plane surface is formed on the outer periphery provided to the projecting shape 81a from the projection section 81b to the shoulder section 81c. The projection section 81b and the shoulder section 81c of the projecting shape 81a and the recessed section 82b and the shoulder section 82c of the recessed shape 82a form the projections and depressions corresponding to each other with respect to the intersecting direction Y.

FIGS. 19 and 20 are perspective views showing the pair of jaw sections 83 and 84.
FIG. 21 is a cross-sectional view showing the pair of jaw sections 83 and 84 disposed correspondingly. It should be noted that the components the same as those of the pair of jaw sections 12 and 13 in the aforementioned first embodiments will be omitted in the explanation. A projection section 83b engages with a recessed section 84b; and a shoulder section 83c engages with a shoulder section 84c as shown in FIG. 21 showing a projecting shape 83a of the jaw sections 83 and a recessed shape 84a of the jaw section 84. In addition, two plane surfaces are formed on the outer periphery provided to the projecting shape 83a from the projection section 83b to the shoulder section 83c. The projection section 83b and the shoulder section 83c of the projecting shape 83a and the recessed section 84b and the shoulder section 84c of the recessed shape 84a form the projections and depressions corresponding to each other with respect to the intersecting direction Y.

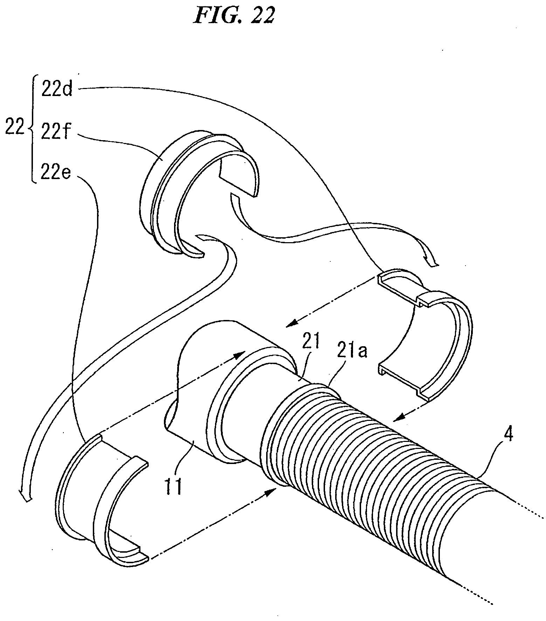

In addition, the rotational ring 22 according to the aforementioned first embodiment is divided longitudinally into two slices 22b and 22c in advance. However, the rotational ring 22 may be formed by two slices 22d and 22e divided longitudinally in advance and a C-letter-shaped stopper 22f for fixing the two slices 22d and 22e joined. Subsequently, manufacturing of the forceps for endoscopic use necessitates after fixing the connecting pipe 21 to the base 11 by brazing method as shown in FIG. 22, assembling the slices 22d and 22e to the outer periphery of the connecting pipe 21, and engaging so that the stopper 22f is opened once and so that the outer periphery of the two slices 22d and 22e are pushed. Subsequently, the inner periphery of the distal end section of the overtube 6 is fixed to the outer periphery of the stopper 22f. It is possible to facilitate the operation for fixing the distal end section of the overtube 6 to the outer periphery of the stopper 22f since the two slices 22d and 22e assembled to the outer periphery of the connecting pipe 21 are fixed once by the stopper 22f. It should be noted that the
first illustrative example can employ the same configuration.

A resin-made sheath may be used in place of the multilayer-single-thread coils, e.g., the three-layer-single-thread coil second sheath 4 of the aforementioned first embodiment, and the three-layer-single-thread coil first sheath 51 of the aforementioned first illustrative example.

In addition, the first sheath 51 disposed inward in the aforementioned first illustrative example may use a multilayer-multithread coil.

In addition, in a modified example of the treatment instrument for endoscopic use according to the second illustrative example, a treatment section may protrude from or retract into the distal end of the sheath having the following configuration. Structural elements that are equivalent to those of the aforementioned second illustrative example in the following explanation will be assigned the same numeric symbols and redundant explanations thereof will be omitted.

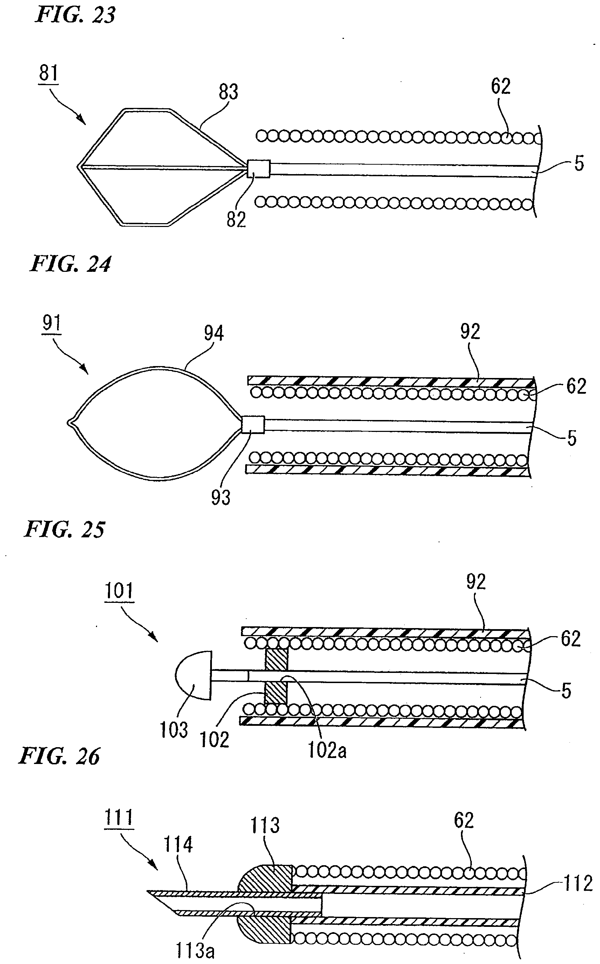

FIG. 23 is a basket 81 provided with a net treatment section 83 connected to the distal end section of the operation wire 5 inserted in the inner periphery of the multithread coil sheath 62 via the connecting member 82. This basket (endoscopic instrument) 81 can remove a calculus from a human body by pushing the operation wire 5, seizing the calculus produced in the human body into the treatment section 83, retracting the operation wire 5, and seizing the calculus in the treatment section 82.

FIG. 24 is a snare (endoscopic instrument) 91 provided with a ring treatment section 94 connected to the distal end section of the operation wire 5 inserted in the inner periphery of the sheath 62 surrounded by a overtube 92 outward in the radial direction via a connecting member 93. This snare 91 can incise tissue by pushing the operation wire 5, surrounding the root of the tissue in the human body by the treatment section 94, and charging electric current supplied by a high frequency electric current-generating
apparatus, which is not shown in the drawing, to the treatment section 94.

Also, FIG. 25 is an incision instrument 101 provided with a base 102 fixed on the inner periphery of the distal end section of the sheath 62 and provided with a hole section 102a; and a treatment section 103 fixed to the distal end section of the operation wire 5 inserted through the hole section 102a. This incision instrument 101 can incise tissue by after inserting into the incision instrument 101 into the human body, pushing the operation wire 5, abutting the treatment section 103 onto an affected site, and charging electric current supplied by the high frequency electric current-generating apparatus which is not shown in the drawing to the treatment section 103.

Also, FIG. 26 shows a syringe instrument (endoscopic instrument) 111 provided with: a base 113 fixed to the distal end section of the sheath 62 and provided with a hole section 113a; a needle treatment section 114 inserted through the hole section 113a; a tube 112 for fixing the proximal end section of the treatment section 114 to the inner periphery of the distal end section; and a pump, not shown in the drawing, fixed in the vicinity of the proximal end of the tube 112. The syringe instrument 111 can move the treatment section 114 and the tube 112 in one unit in the axial direction in one unit. Subsequently, protruding the treatment section 114 retracted relative to the syringe instrument 111 from the base 113 at the affected site upon abutting to the affected site can inject chemicals in the tube 112 by using the pump not shown in the drawing into the affected site.

The present invention is not limited to the above descriptions but is limited only by the appended claims.

## Claims

1. A treatment instrument (1) for endoscopic use comprising:
a cylindrical flexible sheath (2);
an operation wire (5) inserted in a vicinity of an inner periphery side of the sheath (2); and
a cylindrical base (11, 11a, 11b) fixed to a distal end section of the sheath (2), the base (11, 11a, 11b) extending in an axial line direction of the operation wire (5),
wherein
the sheath (2) in full length is formed with a first sheath (3) and a second sheath (4), and the first sheath (3) is disposed inside the second sheath (4), and
a multithread coil is used as at least one of the first sheath (3) and the second sheath (4) of the sheath (2) in full length,
**characterized in that**:
a cylindrical increased diameter section (11a) is formed in the vicinity of a proximal end of the base (11),
a reduced diameter section (11b) is formed more proximally relative to the increased-diameter section (11a),
a distal end section (4a) of the second sheath (4) is fixed between the outer periphery of the reduced diameter section (11b) of the base (11) and the inner periphery of a connecting pipe (21), and
a distal end section (3a) of the first sheath (3) is fixed to a surface in the vicinity of the proximal end of the reduced diameter section (11b) of the base (11).

2. The treatment instrument for endoscopic use according to Claim 1, wherein a multilayer-single-thread coil is used as the second sheath (4).

3. The treatment instrument for endoscopic use according to Claim 1, wherein a multilayer-multithread coil is used as the second sheath (4).

4. The treatment instrument for endoscopic use according to Claim 1, wherein
a multilayer-single-thread coil is used as the first sheath (3); and
a multithread coil is used as the second sheath (4).

5. The treatment instrument for endoscopic use according to Claim 1, wherein
a multilayer-multithread coil is used as the first sheath (3); and
a multithread coil is used as the second sheath (4).

6. The treatment instrument for endoscopic use according to one of Claims 1 to 5, further comprising a treatment section (7) extending from and retracting into a distal end of the sheath (2) by extending or retracting the operation wire (5), the treatment section (7) being connected to a distal end of the operation wire (5).

7. The treatment instrument for endoscopic use according to one of Claims 1 to 5, further comprising: a pair of members (12, 13) supported by the base (11, 11a, 11b) and connected to a distal end section (5a) of the operation wire (5), the pair of members (12, 13) being configured to change a distance between a distal end section of one of the pair of members (12, 13) and a distal end section of another of the pair of members (12, 13) by extending or retracting the operation wire (5).

8. The treatment instrument for endoscopic use according to Claim 7, wherein
the treatment section (7) has a link mechanism (14),
a part of the link mechanism (14) is the pair of members (12, 13), and
projections and depressions corresponding to each other are formed on inner peripheries of the pair of members (12, 13) in the axial line direction of the operation wire (5) and in a direction orthogonal to the axial line direction.

## Patentansprüche

1. Behandlungsinstrument (1) für den endoskopischen Einsatz, umfassend:
eine zylindrische flexible Umhüllung (2);
einen in einer Nähe einer inneren Umfangsseite der Umhüllung (2) eingeführten Bediendraht (5); und
eine zylindrische Basis (11, 11a, 11b), die an einem distalen Endabschnitt der Umhüllung (2) befestigt ist, wobei die Basis (11, 11a, 11b) sich in eine Axiallinienrichtung des Bediendrahts (5) erstreckt,
wobei
die Umhüllung (2) in voller Länge mit einer ersten Umhüllung (3) und einer zweiten Umhüllung (4) geformt ist, und die erste Umhüllung (3) innerhalb der zweiten Umhüllung (4) angeordnet ist, und
eine Mehrfachwicklung, die in voller Länge als die erste Umhüllung (3) und/oder die zweite Umhüllung (4) der Umhüllung (2) verwendet wird,
**dadurch gekennzeichnet, dass**:
ein zylindrischer im Durchmesser vergrößerter Abschnitt (11a) in der Nähe eines proximalen Endes der Basis (11a) geformt ist,
ein im Durchmesser verkleinerter Abschnitt (11b) proximaler relativ zu dem im Durchmesser vergrößerten Abschnitt (11a) geformt ist,
ein distaler Endabschnitt (4a) der zweiten Umhüllung (4) zwischen dem äußeren Umfang des im Durchmesser verkleinerten Abschnitts (11b) der Basis (11) und dem inneren Umfang eines Verbindungsrohrs (21) befestigt ist, und
ein distaler Endabschnitt (3a) der ersten Umhüllung (3) an einer Oberfläche in der Nähe des proximalen Endes des im Durchmesser verkleinerten Abschnitts (11b) der Basis (11) befestigt ist.

2. Behandlungsinstrument für den endoskopischen Einsatz nach Anspruch 1, wobei eine mehrlagige Einfachwicklung als die zweite Umhüllung (4) verwendet wird.

3. Behandlungsinstrument für den endoskopischen Einsatz nach Anspruch 1, wobei eine mehrlagige Mehrfachwicklung als die zweite Umhüllung (4) verwendet wird.

4. Behandlungsinstrument für den endoskopischen Einsatz nach Anspruch 1,
wobei eine mehrlagige Einfachwicklung als die erste Umhüllung (3) verwendet wird; und
eine Mehrfachwicklung als die zweite Umhüllung (4) verwendet wird.

5. Behandlungsinstrument für den endoskopischen Einsatz nach Anspruch 1, wobei
eine mehrlagige Mehrfachwicklung als die erste Umhüllung (3) verwendet wird; und
eine Mehrfachwicklung als die zweite Umhüllung (4) verwendet wird.

6. Behandlungsinstrument für den endoskopischen Einsatz nach einem der Ansprüche 1 bis 5, weiter umfassend einen Behandlungsabschnitt (7), der sich von einem distalen Ende der Umhüllung (2) durch Ausfahren oder Einziehen des Bediendrahts (5) erstreckt und in diese zurückzieht, wobei der Behandlungsabschnitt (7) mit einem distalen Ende des Bediendrahts (5) verbunden ist.

7. Behandlungsinstrument für den endoskopischen Einsatz nach einem der Ansprüche 1 bis 5, weiter umfassend: ein Paar von Elementen (12, 13), das durch die Basis (11, 11a, 11b) gestützt und mit einem distalen Endabschnitt (5a) des Bediendrahts (5) verbunden ist, wobei das Paar von Elementen (12, 13) dazu eingerichtet ist, eine Distanz zwischen einem distalen Endabschnitt eines des Paars von Elementen (12, 13) und einem distalen Endabschnitt eines anderen des Paars von Elementen (12, 13) durch Ausfahren oder Einziehen des Bediendrahts (5) zu ändern.

8. Behandlungsinstrument für den endoskopischen Einsatz nach Anspruch 7, wobei
der Behandlungsabschnitt (7) einen Gelenkmechanismus (14) aufweist,
ein Teil des Gelenkmechanismus (14) das Paar von Elementen (12, 13) ist, und
Vorsprünge und Absenkungen, die sich gegenseitig entsprechen, auf Innenumfängen des Paars von Elementen (12, 13) in der Axiallinienrichtung des Bediendrahts (5) und in eine Richtung gebildet sind, die orthogonal zu der Axiallinienrichtung ist.

## Revendications

1. Instrument de traitement (1) pour utilisation endoscopique comprenant :
une gaine souple cylindrique (2) ;
un câble d'actionnement (5) inséré au voisinage d'un bord périphérique interne de la gaine (2) ; et
une base cylindrique (11, 11a, 11b) fixée à une section d'extrémité distale de la gaine (2), la base (11, 11a, 11b) s'étendant dans une direction de la ligne axiale du câble d'actionnement (5),
dans lequel
la gaine (2) en pleine longueur est formée avec une première gaine (3) et une deuxième gaine (4), et la première gaine (3) est disposée à l'intérieur de la deuxième gaine (4), et
un enroulement multifil est utilisé en tant qu'au moins l'une parmi la première gaine (3) et la deuxième gaine (4) de la gaine (2) en pleine longueur,
**caractérisé en ce que** :
une section à diamètre augmenté cylindrique (11a) est formée au voisinage d'une extrémité proximale de la base (11),
une section à diamètre réduit (11b) est formée de manière plus proximale par rapport à la section à diamètre augmenté (11a),
une section d'extrémité distale (4a) de la deuxième gaine (4) est fixée entre la périphérie externe de la section à diamètre réduit (11b) de la base (11) et la périphérie interne d'un tuyau de raccordement (21), et
une section d'extrémité distale (3a) de la première gaine (3) est fixée à une surface au voisinage de l'extrémité proximale de la section à diamètre réduit (11b) de la base (11).

2. Instrument de traitement pour utilisation endoscopique selon la revendication 1, dans lequel un enroulement à fil unique multicouche est utilisé en tant que deuxième gaine (4).

3. Instrument de traitement pour utilisation endoscopique selon la revendication 1, dans lequel un enroulement multifil multicouche est utilisé en tant que deuxième gaine (4).

4. Instrument de traitement pour utilisation endoscopique selon la revendication 1, dans lequel
un enroulement à fil unique multicouche est utilisé en tant que première gaine (3) ; et
un enroulement multifil est utilisé en tant que deuxième gaine (4).

5. Instrument de traitement pour utilisation endoscopique selon la revendication 1, dans lequel
un enroulement multifil multicouche est utilisé en tant que première gaine (3) ; et
un enroulement multifil est utilisé en tant que deuxième gaine (4).

6. Instrument de traitement pour utilisation endoscopique selon l'une quelconque des revendications 1 à 5, comprenant en outre une section de traitement (7) s'étendant à partir de et se rétractant à l'intérieur d'une extrémité distale de la gaine (2) en étendant ou rétractant le câble d'actionnement (5), la section de traitement (7) étant raccordée à une extrémité distale du câble d'actionnement (5).

7. Instrument de traitement pour utilisation endoscopique selon l'une quelconque des revendications 1 à 5, comprenant en outre : une paire d'éléments (12, 13) supportés par la base (11, 11a, 11b) et raccordés à une section d'extrémité distale (5a) du câble d'actionnement (5), les éléments (12, 13) formant une paire étant configurés pour changer une distance entre une section d'extrémité distale de l'un des éléments (12, 13) formant la paire et une section d'extrémité distale d'un autre des éléments (12, 13) formant la paire en étendant ou rétractant le câble d'actionnement (5).

8. Instrument de traitement pour utilisation endoscopique selon la revendication 7, dans lequel
la section de traitement (7) comporte un mécanisme de liaison (14),
une partie du mécanisme de liaison (14) est la paire d'éléments (12, 13), et des saillies et des creux correspondant les uns avec les autres sont formés sur les périphéries internes des éléments (12, 13) formant une paire dans la direction de la ligne axiale du câble d'actionnement (5) et dans une direction orthogonale à la direction de la ligne axiale.
